# EUROPEAN PATENT APPLICATION

(11) **EP 4 623 893 A1**
(43) Date of publication of application: **01.10.2025**
(21) Application number: 25166362.1
(22) Date of filing: 26.03.2025
(51) Int. Cl.: A61K 8/365, A61K 8/41, A61K 8/44, A61K 8/55, A61K 8/73, A61Q 5/08, A61Q 5/10

(54) **PRETREATMENT COMPOSITION (PC) COMPRISING MALIC ACID AND DTPMP FOR A COLOURING OR BLEACHING PROCESS OF KERATIN FIBERS**

(30) Priority: 26.03.2024 EP 24166313
(71) Applicant: Wella Germany GmbH, 64295 Darmstadt (DE)
(72) Inventor: Forgione, Marianna, 64295 Darmstadt (DE); Gross, Andrej, 64295 Darmstadt (DE); Sitterberg-Muehlthau, Stephanie, 64295 Darmstadt (DE); de Waal, Gabriele, 64295 Darmstadt (DE)
(74) Representative: Ziebig Hengelhaupt Intellectual Property Attorneys Patentanwaltskanzlei PartGmbB

(57) **Abstract**

FIELD OF THE INVENTION

The presently claimed invention relates to a composition (PC) comprising as components malic acid and/or malic acid salt(s) (component (A)), at least one chelant comprising diethylenetriamine penta(methylene phosphonic acid) and/or diethylenetriamine penta(methylene phosphonic acid) salt(s) (component (B)), at least one thickener (component (C)), at least one cosmetically acceptable solvent (component (D)), at least one surfactant (component (E)) and, optionally, at least one care ingredient (component (F)). Another aspect of the presently claimed invention relates to the use of the composition (PC) as a pretreatment composition for a colouring or bleaching process, the use of a composition (PC) for protecting keratin fibers during a colouring or bleaching process and a process for coloring or bleaching keratin fibers, wherein the composition (PC) is applied to keratin fibers. Another aspect of the presently claimed invention relates to a kit comprising the composition (PC) and an oxidative hair colouring composition (HC). Another aspect of the presently claimed invention relates to a multidepartment device comprising a first compartment containing the composition (PC) and a second compartment containing an oxidizing composition.

## Description

### FIELD OF THE INVENTION

The presently claimed invention relates to a composition (PC) comprising as components malic acid and/or malic acid salt(s) (component (A)), at least one chelant comprising diethylenetriamine penta(methylene phosphonic acid) and/or diethylenetriamine penta(methylene phosphonic acid) salt(s) (component (B)), at least one thickener (component (C)), at least one cosmetically acceptable solvent (component (D)), at least one surfactant (component (E)) and, optionally, at least one care ingredient (component (F)), wherein the composition has a pH value in the range of 2.5 to 6.5. Another aspect of the presently claimed invention relates to the use of the composition (PC) as a pretreatment composition for a colouring or bleaching process, the use of a composition (PC) for protecting keratin fibers during a colouring or bleaching process and a process for coloring or bleaching keratin fibers, wherein the composition (PC) is applied to keratin fibers. Another aspect of the presently claimed invention relates to a kit comprising the composition (PC) and an oxidative hair colouring composition (HC). Another aspect of the presently claimed invention relates to a multidepartment device comprising a first compartment containing the composition (PC) and a second compartment containing an oxidizing composition.

### BACKGROUND OF THE INVENTION

It is known practice to perform oxidative hair colouring or bleaching processes to modify the colour of natural keratin fibers, like human hair. For bleaching processes, generally compositions comprising at least one oxidizing agent are used. For oxidative hair colouring processes, generally an oxidative hair colouring composition comprising oxidative dye precursors and at least one oxidizing agent are used. Consumers want to be able to colour their hair to many shades. This requires high lift of hair to deliver a wide range of shades. In order to deliver a wide range of shades, generally a large number of dye precursors are used. Permanent and demi-permanent oxidative hair colouring compositions have been used in professional salons and in retail products for use at home for decades. The oxidative hair colouring compositions typically comprise a so called tint composition and an oxidizing composition, which are typically packaged separately and mixed immediately before use to form the oxidative hair colouring composition. The tint composition contains oxidative dye precursors, typically at least one oxidative primary dye precursor and at least one oxidative coupler dye precursor and an alkalizing agent, which is typically ammonia, an organic amine like monoethanolamine and/or 2-amino-1-propanol. The oxidative primary dye precursors react with the oxidative coupler dye precursor in the presence of the oxidizing agent to form larger, coloured dye products within the hair. The oxidizing composition typically contains a stabilized form of hydrogen peroxide. The oxidizing composition sometimes is referred to as "developer composition".

These oxidative hair colouring or bleaching compositions do have dying or bleaching power, but in some cases, they may be responsible for degradation of the quality of keratin fibers, like human hair. The constituent proteins of the keratin fibers may be denatured, this giving rise to labile proteins. The higher the content of labile proteins in the keratin fibers, the more the keratin fibers are damaged. This degradation of the keratin fibers, like human hair, can result in substantial breakage of the keratin fibers during combing, especially when the oxidative hair colouring compositions or bleaching compositions are applied to sensitized hair.

By consequence, it is common practice to apply care compositions involving conditioning agents in order to limit the degradation of the keratin fibers or to improve the cosmetic properties of the keratin fibers, like human hair. However, these care compositions in some cases impair the oxidative colouring or bleaching of the keratin fibers.

Theres is a need for providing a composition (PC) suitable for being used as a pretreatment composition for protecting keratin fibers during an oxidative colouring process.

### SUMMARY OF THE INVENTION

It is an object of the presently claimed invention to provide a composition (PC) which can be used as a pretreatment composition for a colouring or bleaching process of keratin fibers, preferably for protecting keratin fibers during a colouring or bleaching process.

The object has been solved by A composition (PC) comprising as components.
(A) malic acid and/or malic acid salt(s),
(B) at least one chelant comprising diethylenetriamine penta(methylene phosphonic acid) and/or diethylenetriamine penta(methylene phosphonic acid) salt(s),
(C) at least one thickener,
(D) at least one cosmetically acceptable solvent,
(E) at least one surface active ingredient, and
(F) optionally, at least one care ingredient,
wherein the composition has a pH value in the range of 2.5 to 6.5.

In one embodiment the composition (PC) may comprise as component (G) at least one additional component.

In another aspect, the presently claimed invention is directed to the use a composition (PC) as defined herein as a pretreatment composition for a colouring or bleaching process.

In another aspect, the presently claimed invention is directed to the use of a composition (PC) as defined herein for protecting keratin fibers during a colouring or bleaching process.

In yet another aspect, the presently claimed invention is directed to a process for colouring or bleaching keratin fibers comprising the following steps
i) applying to the keratin fibers a composition (PC) as defined herein,
ii) applying to the keratin fibers a colouring or a bleaching composition.

In yet another aspect, the presently claimed invention is directed to a kit comprising the composition (PC) as defined herein, and the oxidative hair colouring composition (HC) as defined herein, and optionally a conditioner.

In yet another aspect, the presently claimed invention is directed to a multicompartment device comprising
- a first compartment containing the composition (PC) as defined herein,
- a second compartment containing an oxidizing composition comprising as component (HD) at least one oxidizing agent,
- optionally a third compartment containing a tint composition comprising as components (HA) at least one oxidative coupler dye precursor, (HB) at least one oxidative primary dye precursor, and (HC) at least one alkalizing agent, and
- optionally a fourth compartment comprising a conditioner.

Surprisingly, it was found that at least one chelant comprising diethylenetriamine penta(methylene phosophonic acid), and/or salts thereof (component (B)), especially in combination with malic acid, and/or salts thereof (component (A)) can be used in the composition (PC). It was surprisingly found by the inventors of the present invention that the use of the composition (PC) as a pretreatment composition for a colouring or bleaching process can reduce the level of hair damage.

Without being bound to a theory, it is assumed that bi- and trivalent metal cations, like iron cations or copper cations, contained on the surface of the keratin fibers or contained in the water used during the oxidative hair colouring or bleaching process, when contacted with hydrogen peroxide, may lead to the formation of aggressive radicals leading to a high level of hair damage. The chelant (component (B)), especially in combination with malic acid (component (A)) seems to reduce the potency of the formation of aggressive radicals induced by bi- and trivalent metal ions. By consequence, the composition (PC), when used as a pretreatment composition, can lead to a reduction of hair damage in oxidative hair colouring or beaching processes. Surprisingly, it was found out that that the composition (PC) has an improved improved storage stability. The pH value of the composition (PC) does not change, even if the composition (PC) ist stored at elevated temperatures.

### DETAILED DESCRIPTION OF THE INVENTION

The inventors of the present invention surprisingly found that the combination of malic acid and/or malic acid salt(s) (component (A)) and diethylenetriamine penta(methylene phosophonic acid) (component (B)) and/or diethylenetriamine penta(methylene phosophonic acid) salt(s) (component (B)), when used as a pretreatment composition (PC) is effective for protecting keratin fibers in a process for oxidative hair colouring or bleaching.

### Component (A)

In the present case the terms "(A)", "component (A)", and "malic acid, and/or malic acid salt(s)" are used synonymously, and therefore, have preferably the same meaning.

Malic acid is a dicarboxylic acid with the molecular formula C₄H₆O₅. Malic acid has two stereo enantiomeric forms, namely the L- and the D-enantiomer. Only the L-enantiomer exists naturally. Racemic malic acid can be produced by double hydration of maleic anhydride. The racemic malic acid comprises a mixture of the L- and the D-isomer of malic acid.

Malic acid and/or malic acid salt(s) is/are assumed to be one of the active ingredients contained in the composition (PC) for protecting keratin fibers.

As component (A) preferably racemic malic acid and/or salts of racemic malic acid is/are used. Suitable salts of malic acid, preferably racemic malic acid, are sodium or ammonium salts of malic acid, preferably sodium or ammonium salts of racemic malic acid.

In an especially preferred embodiment as component (A) racemic malic acid is used. The CAS number of racemic malic acid is 6915-15-7.

In one embodiment component (A) is present generally in an amount in the range of 0.1 to 2.0 wt.%, preferably in an amount in the range of 0.2 to 1.5 wt.%, particularly preferred in an amount in the range of 0.4 to 1.2 wt.%, in each case based on the total weight of the composition (PC).

All weight amounts (wt.%) given in the present invention in view of component (A) comprised in composition (PC) are in each case preferably calculated based on malic acid in form of the free acid.

### Component (B)

In the present case the terms "(B)", "component (B)", and "at least one chelant comprising diethylenetriamine penta(methylene phosphonic acid) and/or diethylenetriamine penta(methylene phosphonic acid) salt(s)" are used synonymously, and therefore, have preferably the same meaning.

The term "at least one chelant" means exactly one chelant and, also a mixture of two or more different chelants. Preferably a mixture of at least two different chelants is used.

Diethylenetriamine penta(methylene phosphonic acid) is also known under the IUPAC name as {[(Phosphonomethyl)azanediyl]bis[ethane-2,1-diylnitrilobis(methylene)]}tetrakis (phosphonic acid) and has the CAS number 15827-60-8. The abbreviation for diethylenetriamine penta(methylene phosphonic acid) is DTPMP.

DTPMP is normally used as a salt because the acid form has very limited solubility in water and tends to crystallize in concentrated aqueous solutions. Preferably the heptasodium salt of DTPMP (DTPMP.7 Na) is used having the CAS number 22042-96-2.

The wording "diethylenetriamine penta(methylene phosphonic acid) and ethylenediamine-N,N'-disuccinic acid" and "DTPMP" in the present invention includes the free acid as well as salts of DTPMP.

In a preferred embodiment component (B) comprises DTPMP, and ethylenediaminetetraacetic acid and/or ethylenediaminetetraacetic acid salt(s).

The abbreviation for ethylenediaminetetraacetic acid is EDTA. EDTA has the CAS number 60-00-4. EDTA is available as free acid or in the form of several salts, like disodium EDTA, sodium calcium EDTA, and tetrasodium EDTA.

The wording "ethylenediaminetetraacetic acid" and "EDTA" in the present invention includes the free acid as well as salts of EDTA. Preferably the disodium salt of EDTA is used having the CAS number 6381-92-6.

Component (B) comprised in the inventive composition (PC) beside DTPMP, and optionally EDTA in one embodiment may contain one, two or more further chelants selected from the group consisting of hydroxyethyl ethylenediamine triacetic acid (HEDTA), ethylenediamine-N,N'-disuccinic acid (EDDS), ethylenediamine-N,N'-diglutaric acid (EDDG), 2-hydroxypropylenediamine-N,N'-disuccinic acid (HPDDS), N,N-dicarboxymethylglutamic acid (GLDA), glycinamide-N,N'-disuccinic acid (GADS), ethylenediamine-N-N'-bis(ortho-hydroxyphenyl acetic acid) (EDDHA), dimethyl glucamine (DMG), N-(1-carboxyethyl)iminodiacetic acid, methylglycine N,N-diacetic acid (MGDA), amino trimethylene phosphonic acid (ATMP), and salts thereof.

All weight amounts (wt.%) given in the present invention in view of chelants comprised in component (B) are in each case calculated on basis of the chelants in form of the free acid.

In other words, if the amount of DTPMP comprised in the oxidative colouring composition in wt.% is given, the amount of DTPMP in wt.% is calculated based on DTPMP in form of a free acid and not based on DTPMP in form of a salt. The same holds true for EDTA and any other chelant that might be comprised in the composition (PC).

In a preferred embodiment composition (PC) comprises component (B) generally in an amount in the range from ≥ 0.1 wt.% to ≤ 1.5 wt.%, preferably in an amount in the range of 0.2 to 1.0 wt.%, particularly preferred in an amount in the range of 0.4 to 0.8 wt.%, based on the total weight of composition (PC).

The amount of the component (B) at present means the amount of all chelants comprised in the component (B), preferably comprised composition (PC).

In an especially preferred embodiment component (B) comprises a mixture of
0.2 to 1.4 wt.% of DTPMP, and
0.05 to 0,5 wt. % of EDTA,
wherein the wt.% values are based on the total weight of composition (PC).

In a particularly preferred embodiment component (B) comprises a mixture of
0.5 to 1.0 wt.% of DTPMP, and
0.1 to 0.2 wt. % of EDTA,
wherein the wt.% values are based on the total weight of composition (PC).

In a preferred embodiment component (B) beside DTPMP, and optionally EDTA contains less than 10 wt.%, more preferred less than 5 wt.% of further chelant, based on the total weight of component (B) comprised in composition (PC), preferably based on the total weight of composition (PC).

In a more preferred embodiment component (B) beside DTPMP, and optionally EDTA does not contain a further chelant. In another preferred embodiment composition (PC) beside DTPMP, and optionally EDTA does not contain a further chelant.

### Component (C)

In the present case the terms "(C)", "component (C)" and "at least one thickener" are used synonymously, and therefore have preferably the same meaning. The term "at least one thickener" means exactly one thickener and, also a mixture of two or more different thickeners.

The composition (PC) of the presently claimed invention preferably comprises at least one thickener, in particular a polymeric thickener in an amount that is sufficient to impart a viscosity to the composition (PC) that allows for its ready application to hair without unduly dripping off the hair, as is known in the art.

In a preferred embodiment composition (PC) comprises as component (C) at least one water soluble polymer, preferably selected from the group consisting of cellulose-based polymers, polysaccharide-based polymers, and acrylate-based polymers.

In a preferred embodiment composition (PC) comprises component (C) generally in an amount in the range from ≥ 0.1 wt.% to ≤ 5.0 wt.%, preferably in an amount in the range of 0.1 to 3.0 wt.%, particularly preferred in an amount in the range of 0.1 to 2.5 wt.%, based on the total weight of composition (PC).

Examples of commonly used polymeric thickeners are sold under the tradename Aculyn-22 and Aculyn-33 by the company Rohm & Haas, Permulen TR1, Carbopol 2020, Carbopol Ultrez-21 by the company Noveon, and Structure 2001 and Structure 3001 by the company National Starch. Other suitable polymeric thickeners include polyether polyurethanes, for example Aculyn-44 and Aculyn-46 by the company Rohm and Haas. Another suitable polymeric thickener is cellulose modified with groups comprising at least one C8 - C30 fatty chain, such as the product Natrosol Plus Grade 330 CS sold by the company Aqualon.

In one embodiment of the presently claimed invention the composition (PC) comprises salt tolerant thickeners, including but not limited to: xanthan, guar, hydroxypropyl guar, scleroglucan, methyl cellulose, ethyl cellulose (available as AQUACOTE^{®}, hydroxyethyl cellulose (NATROSOL^{®}), carboxymethyl cellulose, hydroxypropylmethyl cellulose, microcrystalline cellulose, hydroxybutylmethyl cellulose, hydroxypropyl cellulose (available as KLUCEL^{®}), hydroxyethyl ethyl cellulose, cetyl hydroxyethyl cellulose (available as NATROSOL^{®} Plus 330), N-vinylpyrrolidone (available as POVIDONE^{®}), Acrylates/Ceteth-20 Itaconate Copolymer (available as STRUCTURE^{®} 3001), hydroxypropyl starch phosphate (available as STRUCTURE^{®} ZEA), polyethoxylated urethanes or polycarbamyl polyglycol ester (e.g. PEG-150/Decyl/SMDI copolymer (e.g. ACULYN^{®} 44), PEG- 150/Stearyl/SMDI copolymer (available as ACULYN^{®} 46), trihydroxystearin (available as THIXCIN^{®}), acrylates copolymer (e.g. available as ACULYN^{®} 33) or hydrophobically modified acrylate copolymers (e.g. Acrylates / Steareth-20 Methacrylate Copolymer (available as ACULYN^{®} 22), acrylates/steareth-20 methacrylate crosspolymer (available as ACULYN^{®} 88), acrylates/vinyl neodecanoate crosspolymer (available as ACULYN^{®} 38), acrylates/beheneth-25 methacrylate copolymer (available as ACULYN^{®} 28), acrylates/C 10-30 alkyl acrylate crosspolymer (available as Carbopol^{®} ETD 2020), non-ionic amphophilic polymers comprising at least one fatty chain and at least one hydrophilic unit selected from polyether urethanes comprising at least one fatty chain.

Suitable cellulose-based polymers are for example selected from the group consisting of methyl cellulose, ethyl cellulose, hydroxyethyl cellulose, carboxymethyl cellulose, hydroxypropylmethyl cellulose, microcrystalline cellulose, hydroxybutylmethyl cellulose, hydroxypropyl cellulose, hydroxyethyl ethyl cellulose, and cetyl hydroxyethyl cellulose.

Preferred cellulose-based polymers are selected from the group consisting of hydroxyethyl cellulose.

Suitable polysaccharide-based polymers are for example selected from the group consisting of xanthan, guar, hydroxypropyl guar, scleroglucan, and hydroxypropyl starch phosphate.

Preferred polysaccharide-based polymers are selected from the group consisting of xanthan, guar, hydroxypropyl guar, and hydroxypropyl starch phosphate.

Suitable acrylate-based polymers are for example selected from the group consisting of acrylates copolymer, hydrophobically modified acrylate copolymers, acrylates/steareth-20 methacrylate crosspolymer, acrylates/vinyl neodecanoate crosspolymer, acrylates/beheneth-25 methacrylate copolymer, and acrylates/C 10-30 alkyl acrylate crosspolymer.

Preferred acrylate-based polymers are selected from the group consisting of acrylates copolymer, acrylates/steareth-20 methacrylate crosspolymer, and acrylates/C 10-30 alkyl acrylate crosspolymer.

### Component (D)

In the present case the terms "(D)", "component (D)" and "at least one cosmetically acceptable solvent" are used synonymously, and therefore have preferably the same meaning. The term "at least one cosmetically acceptable solvent" means exactly one cosmetically acceptable solvent and, also a mixture of two or more different cosmetically acceptable solvents. Preferably as component (D) a mixture of two or more different cosmetically acceptable solvents is used.

The cosmetically acceptable solvent (component (D)) used in the composition (PC), preferably is water-based, alcohol-based or water-alcohol-based. Typically, the cosmetically acceptable solvent comprises water and one or more monovalent and/or polyvalent C1-C4 alcohols. According to embodiments, component (D) comprises water and at least one alcohol selected from the group consisting of ethanol, n-propanol, isopropanol, glycerol, ethylene glycol, propylene glycol, butylene glycol and ethoxdiglycol.

In one embodiment component (D) is generally present in an amount in the range of 71.75 to 98.7 wt.%, preferably in an amount in the range of 75.5 to 97.15 wt.%, particular preferred in an amount in the range of 79.5 to 96.4 wt.%, in each case based on the total weight of the composition (PC).

In a preferred embodiment component (D) comprises at least 50 % by weight, more preferred at least 60 % by weight, and particularly preferred at least 80 % by weight of water, based on the total weight of component (D) comprised in the composition (PC).

### Component (E)

In the present case the terms "(E)", "component (E)" and "at least one surfactant" are used synonymously, and therefore preferably have the same meaning. The term "at least one surfactant" means exactly one surfactant and, also a mixture of two or more different surfactants.

The surfactants suitable as component (E) include anionic, cationic, amphoteric and non-ionic surfactants.

The anionic surfactants may be selected from the group consisting of salts (such as alkaline salts, for example, sodium salts, ammonium salts, amine salts, amino alcohol salts and magnesium salts) of the following compounds: alkyl sulphates, alkyl ether sulphates, alkylamido ether sulphates, alkylarylpolyether sulphates, monoglyceride sulphates; alkyl sulphonates, alkyl phosphates, alkylamide sulphonates, alkylaryl sulphonates, alpha-olefin sulphonates, paraffin sulphonates; alkyl sulphosuccinates, alkyl ether sulphosuccinates, alkylamide sulphosuccinates; alkyl sulphosuccinamates; alkyl sulphoacetates; alkyl ether phosphates; acyl sarcosinates; acyl isethionates; N-acyltaurates; and mixtures thereof. The alkyl or acyl radical of all of these various compounds, for example, comprises from 8 to 24 carbon atoms, and the aryl radical, for example, is chosen from phenyl and benzyl groups. Weakly anionic surfactants can also be used, such as alkyl-D-galactosiduronic acids and their salts, as well as polyoxyalkylenated (C6-C24) alkyl ether carboxylic acids, polyoxyalkylenated (C6-C24) alkylaryl ether carboxylic acids, polyoxyalkylenated (C6-C24) alkylamido ether carboxylic acids and their salts, for example, those comprising from 2 to 50 ethylene oxide groups, and mixtures thereof. Anionic derivatives of polysaccharides, for example carboxyalkyl ether of alkyl polyglucosides, can be also used.

Suitable anionic surfactant(s) (Component (E)) may comprise at least one anionic functional group at their head selected from sulfate, sulfonate, and phosphate.

Suitable alkyl sulfates include ammonium lauryl sulfate, sodium lauryl sulfate (sodium dodecyl sulfate, SLS, or SDS), and alkyl-ether sulfates, such as sodium laureth sulfate (sodium lauryl ether sulfate or SLES), and sodium myreth sulfate. Further suitable anionic surfactants may include docusate (dioctyl sodium sulfosuccinate), alkyl-aryl ether phosphate, alkyl ether phosphate, sodium lauroyl sarcosinate, ammonium laureth sulfate, disodium lauryl sulfosuccinate, and sodium lauryl sulphoacetate.

Preferred anionic surfactants may be selected from the group consisting of sodium laurylethersulfate, sodium laurethethersulfate, sodium dodecyl sulfate, ammonium laurethethersulfat, ammonium dodecyl sulfate, alkylbenzenesulfonate, and combinations thereof.

The surfactant (component (E)) may be a non-ionic surfactant. The non-ionic surfactant may be selected from the group consisting of lanolin alcohol, and polyoxyethylene ethers of fatty alcohols, and mixtures thereof. The non-ionic surfactant may be preferably ceteareth-n, wherein n is from 2 to 100, or from 10 to 30. Suitable nonionic surfactants are compounds that are well known (see, for example, in this respect "Handbook of Surfactants" by M. R. Porter, published by Blackie & Son (Glasgow and London), 1991, pp. 116-178). Preferred surfactants encompass non-ionic surfactants, in particular polyoxyethylated fatty alcohols or oils. Particularly preferred non-ionic surfactants include polyoxyethylated Castor Oil and hydrogenated polyoxylated Castor Oil. Specific examples of non-ionic surfactants that may be used in the compositions of the present disclosure are PEG-35 Castor Oil, PEG-40 Hydrogenated Castor Oil, and combinations thereof.

Component (E) is preferably present in an amount of from 1.0 to 5.0 % by weight, more preferably in an amount of from 1.25 to 4.0 % by weight, and particularly preferred in an amount of from 1.5 to 3.0 % by weight, wherein the % by weight values are based on the total weight of the composition (PC).

### Component (F)

In the present case the terms "(F)", "component (F)" and "at least one care ingredient" are used synonymously, and therefore have preferably the same meaning. The term "at least one care ingredient" means exactly one care ingredient and, also a mixture of two or more different care ingredients.

Component (F) is preferably present in an amount of from 0 to 5.0 % by weight, more preferably in an amount of from 1.0 to 5.0 % by weight, and particularly preferred in an amount of from 1.0 to 3.0 % by weight, wherein the % by weight values are based on the total weight of the composition (PC).

In a preferred embodiment component (F) is at least one care ingredient selected form the group consisting of quaternary ammonium compounds, fatty compounds, and silicone compounds.

Suitable quaternary ammonium compounds in one embodiment are one or more selected from the group consisting of Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-42, Polyquaternium-43, Polyquaternium-44, Polyquaternium-45, Polyquaternium-46, and Polyquaternium-47, and cetrimonium chloride, behentrimonium chloride, dipalmitoylethylhydroxy ethylmonium methosulfate, behentrimonium methosulfate, and mixtures thereof.

In one embodiment, the fatty compound is preferably selected from the group consisting of fatty alcohols, fatty acids, fatty alcohol derivatives and fatty acid derivatives.

In one embodiment, the fatty alcohols preferably have from 14 to 30 carbon atoms, more preferably from 16 to 22 carbon atoms and can be linear or branched; more preferably the fatty alcohols are selected from the group consisting of cetyl alcohol, stearyl alcohol and behenyl alcohol.

In one embodiment, the fatty acids preferably have from 10 to 30 carbon atoms, more preferably from 12 to 22 carbon atoms, and more preferably from 16 to 22 carbon atoms and can be linear or branched. Also included herein are salts of these fatty acids. More preferably, the fatty acids are selected from the group consisting of lauric acid, palmitic acid, stearic acid, behenic acid and sebacic acid.

In one embodiment, the fatty alcohol derivatives and the fatty acid derivatives are prefera-bly selected from the group consisting of alkyl ethers of fatty alcohols, alkoxylated fatty alcohols, alkyl ethers of alkoxylated fatty alcohols, esters of fatty alcohols, fatty acid esters of compounds having esterifiable hydroxy groups and hydroxy-substituted fatty acids. More preferably, the fatty alcohol derivatives and fatty acid derivatives are selected from the group consisting of methyl stearyl ether; the ceteth series of compounds such as ceteth-1 through ceteth-45, which are ethylene glycol ethers of cetyl alcohol, wherein the numeric designation indicates the number of ethylene glycol moieties present; the stea-reth series of compounds such as steareth-1 through 10, which are ethylene glycol ethers of steareth alcohol, wherein the numeric designation indicates the number of ethylene glycol moieties present; ceteareth-1 through ceteareth-10, which are the eth-ylene glycol ethers of ceteareth alcohol, i.e. a mixture of fatty alcohols containing predomi-nantly cetyl and stearyl alcohol, wherein the numeric designation indicates the number of ethylene glycol moieties present; C1-C30 alkyl ethers of the ceteth, steareth, and ce-teareth compounds; polyoxyethylene ethers of behenyl alcohol; ethyl stearate, cetyl stea-rate, cetyl palmitate, stearyl stearate, myristyl myristate, polyoxyethylene cetyl ether stea-rate, polyoxyethylene stearyl ether stearate, polyoxyethylene lauryl ether stearate, eth-ylene glycol monostearate, polyoxyethylene monostearate, polyoxyethylene distearate, propyleneglycol monostearate, propyleneglycol distearate, trimethylolpropane distearate, sorbitan stearate, polyglyceryl stearate, glyceryl monostearate, glyceryl distearate and glyceryl tristearate.

Suitable silicone compounds are one or more selected form the group consisting of polydimethylsiloxanes, polydiethylsiloxanes and polymethylphenylsiloxanes, wherein polydimethylsiloxanes are preferred.

### Component (G)

In the present case the terms "(G)", "component (G)" and "at least one additional component" are used synonymously, and therefore have preferably the same meaning. The term "at least one additional component" means exactly one additional component and, also a mixture of two or more different additional components. Preferably as component (G) a mixture of two, three or more different additional components is used.

Component (G) is preferably present in an amount of from 0 to 10.0 % by weight, more preferably in an amount of from 0.1 to 10.0 % by weight, and particularly preferred in an amount of from 0.2 to 10.0 % by weight, wherein the % by weight values are based on the total weight of the composition (PC).

Composition (PC) optionally may comprise as component (G) at least one cosmetically acceptable additive, selected from the group consisting of perfumes/fragrances, opacifiers, preservatives, pH modifiers, , physiologically compatible silicone derivative compounds, light protecting agents, anti-flaking agents, hair luster-imparting agents, combability improving agents, defatting agents, moisturizers, cooling agents. The cosmetically acceptable additives may be used in any combinations, as desired or required.

Suitable cosmetically acceptable additives not specifically described below are listed in the International Cosmetics Ingredient Dictionary and Handbook, (8th ed.; The Cosmetics, Toiletry, and Fragrance Association). Particularly, vol. 2, sections 3 (Chemical Classes) and 4 (Functions), which are useful in identifying specific additives to achieve a particular purpose or multipurpose. A few of these additives are discussed below, whose disclosure is of course non-exhaustive.

Suitable amount ranges for perfumes, opacifiers, preservatives, pH modifiers, physiologically compatible silicone derivative compounds, light protecting agents, anti-flaking agents, hair luster-imparting agents, combability improving agents, defatting agents, moisturizers, cooling agents are indicated for additives discussed below. These cosmetically acceptable additives may be present in a total amount of up to 10.0, in particular up to 7.0 percent-by-weight, based on the weight of composition (PC). Typically, the total amount of these cosmetically acceptable additives may be within a range of from 0.010 to 10.0, conveniently within a range of from 0.010 to 7.0 percent-by-weight, based on the weight of composition (PC). For example, the total amount of these cosmetically acceptable additives may be within a range of from 0.010 to 5.0, conveniently within a range of from 0.010 to 3.0 percent-by-weight, based on the weight of composition (PC).

Composition (PC) used according to the present disclosure may comprise a perfume/fragrance. For example, composition (PC) may comprise from 0.0010 to 1.50, in particular 0.010-0.50 percent-by-weight perfume/fragrance, based on the weight of composition (PC). Perfume/fragrance can provide an enhanced user experience by making composition (PC) smell pleasant and/or invoke emotions, such as relaxing or exciting smells.

Alternatively, composition (PC) used according to the present disclosure may be substantially free of perfume and/or fragrance. Some consumers prefer perfume-free compositions. The perfume/fragrance may be an animal fragrance or a plant fragrance. The animal fragrance may be selected from the group consisting of musk oil, civet, castoreum, ambergris, and mixtures thereof.

The plant fragrance may be selected from the group consisting of nutmeg extract, cardomon extract, ginger extract, cinnamon extract, patchouli oil, geranium oil, orange oil, mandarin oil, orange flower extract, cedarwood, vetyver, lavandin, ylang extract, tuberose extract, sandalwood oil, bergamot oil, rosemary oil, spearmint oil, peppermint oil, lemon oil, lavender oil, citronella oil, chamomille oil, clove oil, sage oil, neroli oil, labdanum oil, eucalyptus oil, verbena oil, mimosa extract, narcissus extract, carrot seed extract, jasmine extract, olibanum extract, rose extract, and mixtures thereof.

The perfume may comprise one or more scents selected from the group consisting of acetophenone, adoxal, aldehyde C-12, aldehyde C-14, aldehyde C-18, allyl caprylate, ambroxan, amyl acetate, dimethylindane derivatives, α-amylcinnamic aldehyde, anethole, anisaldehyde, benzaldehyde, benzyl acetate, benzyl alcohol and ester derivatives, benzyl propionate, benzyl salicylate, borneol, butyl acetate, camphor, carbitol, cinnamaldehyde, cinnamyl acetate, cinnamyl alcohol, cis-3-hexanol and ester derivatives, cis-3-hexenyl methyl carbonate, citral, citronnellol and ester derivatives, cumin aldehyde, cyclamen aldehyde, cyclo galbanate, damascones, decalactone, decanol, estragole, dihydromyrcenol, dimethyl benzyl carbinol, 6,8-dimethyl-2-nonanol, dimethyl benzyl carbinyl butyrate, ethyl acetate, ethyl isobutyrate, ethyl butyrate, ethyl propionate, ethyl caprylate, ethyl cinnamate, ethyl hexanoate, ethyl valerate, ethyl vanillin, eugenol, exaltolide, fenchone, fruity esters such as ethyl 2-methyl butyrate, galaxolide, geraniol and ester derivatives, helional, 2-heptonone, hexenol, α-hexylcinnamic aldehyde, hydroxycitrolnellal, indole, isoamyl acetate, isoeugenol acetate, ionones, isoeugenol, isoamyl iso-valerate, iso E super, limonene, linalool, lilial, linalyl acetate, lyral, majantol, mayol, melonal, menthol, p-methylacetophenone, methyl anthranilate, methyl cedrylone, methyl dihydrojasmonate, methyl eugenol, methyl ionone, methyl-α-naphthyl ketone, methylphenylcarbinyl acetate, mugetanol, γ-nonalactone, octanal, phenyl ethyl acetate, phenyl-acetaldehyde dimethyl acetate, phenoxyethyl isobutyrate, phenyl ethyl alcohol, pinenes, sandalore, santalol, stemone, thymol, terpenes, triplal, triethyl citrate, 3,3,5-trimethylcyclohexanol, γ-undecalactone, undecenal, vanillin, veloutone, verdox, and mixtures thereof.

Composition (PC) according to with the present disclosure may comprise a preservative or mixture of preservatives. Compositions (PC) may comprise the preservative(s) in an amount of up to 1.0, for example from 0.0010 to 1.0, in particular from 0.010 to 0.90 percent-by-weight, based on the weight of composition (PC). Cosmetically acceptable preservatives include organic acids such as para-hydroxybenzoic acid; organic acid salts such as sodium benzoate; compounds such as benzyl alcohol, phenoxyethanol, 1,3-bis(hydroxymethyl)-5,5-dimethylimidazolidine-2,4-dione; capryl glycol, and mixtures thereof.

Composition (PC) according to the present disclosure may comprise a pH modifier and/or buffering agent, in a sufficient amount to effectively adjust the pH of the composition. For example, the compositions may comprise up to 1.0, for example from 0.000010 to 1.0, in particular 0.0010-0.50 percent-by-weight pH modifiers, based on the weight of the composition (PC). Suitable pH modifiers and/or buffering agents for use herein include, but are not limited to: and guanidium salts, alkali metal and ammonium hydroxides and carbonates, such as sodium hydroxide, sodium silicate, sodium meta silicate and ammonium carbonate, ammonium bicarbonate, ammonium chloride, and acids such as organic and inorganic acids, for example ascorbic acid, citric acid, acetic acid or tartaric acid, phosphoric acid, hydrochloric acid, and mixtures thereof.

### Use of the composition (PC) / Process for colouring or bleaching

The inventive composition can be advantageously used as a pretreatment composition for a colouring or bleaching process for keratin fibers, preferably human hair.

The inventive composition (PC), moreover can be used for protecting keratin fibers, preferably human hair, during a colouring or bleaching process.

The composition (PC), after applying to the keratin fibers, preferably to the human hair, preferably is left on the keratin fibers, preferably on the human hair, for a time period in the range of 1 to 45 minutes, preferably 2 to 20 minutes and especially preferred 4 to 12 minutes.

Another aspect of the present invention is a process for colouring or bleaching keratin fibers comprising the following steps.
i) applying to the keratin fibers a composition (PC) as defined herein,
ii) applying to the keratin fibers a colouring or a bleaching composition.

In a preferred embodiment the colouring or the bleaching composition used in step ii) comprises at least one oxidizing agent (HD) selected from the group consisting of hydrogen peroxide, urea peroxide, alkali metal bromates, peroxygenated salts, peracids, percarbonates, persulfates and precursors thereof, and mixtures thereof.

In another preferred embodiment during step ii) an oxidative hair colouring composition (HC) is applied comprising as components
(HA) at least one oxidative coupler dye precursor,
(HB) at least one oxidative primary dye precursor,
(HC) at least one alkalizing agent, and
(HD) at least one oxidizing agent.

The oxidizing agent (HD) is preferably as described above.

In an especially preferred the composition (PC) applied in step i) the is left on the keratin fibers for a time ranging from 1 to 60 min, preferably for a time ranging from 2 to 30 min.

The composition (PC) is preferably used as a leave on composition. The term "leave on" preferably means that the composition (PC) is not rinsed off between step i) and step ii). In another preferred embodiment the process for coluring or bleaching keratin fibers does not comprise a rinsing step between step i) and step ii).

Another object of the present invention is a kit comprising the composition (PC) as defined herein, and the oxidative hair colouring composition (HC) as defined herein, and optionally a conditioner.

In a preferred embodiment the kit comprises the oxidative hair colouring composition (HC) in form of a tint composition comprising components (HA), (HB), and (HC), and an oxidizing composition comprising component (HD), wherein the tint composition and the oxidizing composition can be mixed together to obtain the hair colouring composition (HC).

Another object of the present invention is a multi compartment device comprising
- a first compartment containing the composition (PC) as defined herein;
- a second compartment containing an oxidizing composition comprising as component (HD) at least one oxidizing agent,
- optionally a third compartment containing a tint composition comprising as components (HA) at least one oxidative coupler dye precursor, (HB) at least one oxidative primary dye precursor, and (HC) at least one alkalizing agent, and
- optionally a fourth compartment comprising a conditioner.

For the at least one oxidative coupler dye precursor (HA), the at least one oxidative primary dye precursor (HB), the at least one alkalizing agent (HC), and the at least one oxidizing agent (HD) commonly known components in the field of hair colouring and hair bleaching can be used.

Suitable oxidizing agents (HD) are described above, wherein hydrogen peroxide is especially preferred.

In one embodiment, the at least one alkalizing agent (HC) is at least one alkalizing agent selected from the group consisting of ammonia and alkanolamines. In one embodiment, the alkanolamines are selected from the group consisting of monoethanolamine, propanolamine, isopropanolamine, 2-amino-2-methyl-1-propanol, 2-amino-1-propanol, 2-amino-1-butanol, 1-amino-2-propanol, 3-amino-1-propanol, 2-aminopropane-1,3-diol, 1-amino-2-butanol, 4-amino-2-butanol, 4-amino-1-butanol, 3-amino-1-butanol, 3-amino-2-butanol, 2-amino-1-butanol, dimethyl glucamine, 2,5-diaminocyclohexane-1,4-diol, 2-aminocycohexan-1-ol, 2,4,6-triaminocyclohexane-1,3,5-triol, 2-amino-2-methyl-1,3-popanediol, 2-amino-1-hydroxylmethyl-1,3-propanediol and 2-dimethylamino-2-methyl-1-propanol. In a preferred embodiment, the at least one alkalizing agent (A) is selected from the group consisting of ammonia, monoethanolamine, propanolamine, isopropanolamine and 2-amino-1-propanol; more preferably the at least one alkalizing agent (A) is monoethanolamine and/or ammonia.

In one embodiment of the at least one oxidative primary dye precursors (HB), is at least one oxidative primary dye precursors selected from of the group consisting of toluene-2,5-diamine, p-phenylenediamine, n-phenyl-p-phenylenediamine, N,N-bis(2-hydroxyethyl)-p-phenylenediamine, hydroxyethyl-p-phenylenediamine sulphate, hydroxypropyl bis(n-hydroxyethyl-p-phenylenediamine), 2-methoxymethyl-p-phenylenediamine, 2-chloro-p-phenylenediamine, p-aminophenol, p-methylaminophenol, 4-amino-m-cresol, 6-amino-m-cresol, bis(5-amino-2-hydroxyphenyl)methane, tetraaminopyrimidine, 2,5,6-triamino-4-pyrimidinol, 4,5-diamino-1-(2-hydroxyethyl)pyrazol 2,3-diaminodihydroxypyrazolopyrazolone dimethosulfonate, 4,5-diamino-1-hexylpyrazol, hydroxypropyl-p-phenylenediamine, dimethylpiperazinium aminopyrazolopyridine chloride hydrochloride, methylimidazoliumpropyl p-phenylenediamine, hydroxyethoxy aminopyrazolopyridine and salts thereof.

**In** one embodiment the at least one oxidative coupler dye precursors (HA) is at least one oxidative coupler dye precursors selected from the group consisting of resorcinol, 4-chlororesorcinol, 2-chlororesorcinol, 2-methylresorcinol, m-aminophenol, 4-amino-2-hydroxytoluene, 2-methyl-5-hydroxyethylaminophenol, 3-amino-2,6-dimethylphenol, 3-amino-2,4-dichlorophenol, 5-amino-6-chloro-o-cresol, 5-amino-4-chloro-o-cresol, hydroxybenzo-morpholine, 2-amino-5-ethylphenol, 6-amino-m-cresol, 6-amino-o-cresol, 2,4-diaminophenoxyethanol, 2-amino-4-hydroxyethylaminoanisole, 1,3-bis-(2,4-diamino-phenoxy)propane, 2,6-dihydroxyethylaminotolueneµ, m-phenylenediamine, 2,4-diamino-1,5-di(2-hydroxyethoxybenzene, 1-naphthol, 2-methyl-1-naphthol, 1,5-naphthalenediol, 2,7-naphthalenediol, 1-acetoxy-2-methylnaphthalene, 2,6-dihydroxy-3,4-dimethylpyridine, 2,6-dimethoxy-3,5-pyridinediamine, 3-amino-6-methoxy-2-(methylamino)-pyridine, 2-amino-3-hydroxypyridine, 2,6-diaminopyridine, dihydroxy-indole, 6-hydroxyindole, dihydroxyindoline, phenyl methyl pyrazolone, 1,2,4-trihydroxybenzene, 5-((2-hydroxyethyl)amino)-1,3-benzodioxol, isatin, hydroquinone, 4-formyl-1-methylquinolinium-p-toluenesulfonate, resveratrol and salts thereof.

These and other primary dye precursors (F) and coupler dye precursors (G) may be used in different combination to achieve the nuance sought, as is known in the art.

### Embodiments

**In** the following, there is provided a list of embodiments to further illustrate the present disclosure without intending to limit the disclosure to the specific embodiments listed below.
1. A composition (PC) comprising as components
   (A) malic acid and/or malic acid salt(s),
   (B) at least one chelant comprising diethylenetriamine penta(methylene phosphonic acid) and/or diethylenetriamine penta(methylene phosphonic acid) salt(s),
   (C) at least one thickener,
   (D) at least one cosmetically acceptable solvent,
   (E) at least one surface active ingredient, and
   (F) optionally, at least one care ingredient,
   wherein the composition has a pH value in the range of 2.5 to 6.5.
2. The composition (PC) according to embodiment 1, comprising
   component (A) in an amount of from 0.1 to 2 % by weight,
   component (B) in an amount of from 0.1 to 1.5 % by weight,
   component (C) in an amount of from 0.1 to 5 % by weight, and
   component (D) in an amount of from 71.75 to 98.7 % by weight,
   component (E) in an amount of from 1 to 5 % by weight,
   component (F) in an amount of from 0 to 5 % by weight,
   wherein the % by weight values are based on the total weight of the composition (PC).
3. The composition (PC) according to embodiment 1 or 2, wherein the composition (PC) has a pH value in the range of 2.5 to 6.5.
4. The composition (PC) according to any of embodiments 1 to 3, wherein the composition (PC) has a viscosity in the range of 1000 to 2000 mPas, measured on a Brookfield RS viscosimeter, cone C75, shear rate 64.5 per second, at 25 °C, 30 seconds spin time.
5. The composition (PC) according to any of embodiments 1 to 4, wherein component (C) is at least one water soluble polymer preferably selected from the group consisting of cellulose-based polymers, polysaccharide-based polymers, and acrylate-based polymers.
6. The use of a composition (PC) as defined in any of embodiments 1 to 5, as a pretreatment composition for a colouring or bleaching process.
7. The use of a composition (PC) as defined in any of embodiments 1 to 5, for protecting keratin fibers during a colouring or bleaching process.
8. A process for colouring or bleaching keratin fibers comprising the following steps
   i) applying to the keratin fibers a composition (PC) as defined in any of embodiments 1 to 5,
   ii) applying to the keratin fibers a colouring or a bleaching composition.
9. The process according to embodiment 8, wherein the colouring or the bleaching composition comprises at least one oxidizing agent (HD) selected from the group consisting of hydrogen peroxide, urea peroxide, alkali metal bromates, peroxygenated salts, peracids, percarbonates, persulfates and precursors thereof, and mixtures thereof.
10. The process according to embodiment 8 or 9, wherein the composition applied during step ii) is an oxidative hair colouring composition (HC) comprising as components
   (HA) at least one oxidative coupler dye precursor,
   (HB) at least one oxidative primary dye precursor,
   (HC) at least one alkalizing agent, and
   (HD) at least one oxidizing agent.
11. The process according to any of embodiments 8 to 10, wherein in step i) the composition (PC) is left on the keratin fibers for a time ranging from 1 to 60 min.
12. The process according to any of embodiments 8 to 11, wherein the process does not comprise a rinsing step between step i) and step ii).
13. A kit comprising the composition (PC) according to any of embodiments 1 to 5, and the oxidative hair colouring composition (HC) as defined in embodiment, and optionally a conditioner.
14. The kit according to embodiment 13, wherein the kit comprises the oxidative hair colouring composition (HC) in form of a tint composition comprising components (HA), (HB), and (HC), and an oxidizing composition comprising component (HD), wherein the tint composition and the oxidizing composition can be mixed together to obtain the hair colouring composition (HC).
15. A multi compartment device comprising
   - a first compartment containing the composition (PC) according to any of embodiments 1 to 5;
   - a second compartment containing an oxidizing composition comprising as component (HD) at least one oxidizing agent,
   - optionally a third compartment containing a tint composition comprising as components (HA) at least one oxidative coupler dye precursor, (HB) at least one oxidative primary dye precursor, and (HC) at least one alkalizing agent, and
   - optionally a fourth compartment comprising a conditioner.

### Examples

The following examples illustrate the formulations and performance results according to the presently claimed invention. These examples are not intended to be inclusive of all aspects of the subject matter disclosed herein, but rather to illustrate representative compositions and results. These examples are not intended to exclude equivalents and variations of the presently claimed invention, which are apparent to one skilled in the art.

DSC measurements of hair were conducted. The DSC measurements serve to determine the denaturation temperature of the alpha-helices of the hair. A higher denaturation temperature is related to a higher cross-linking of the matrix (e.g. higher cystine content). The more cross-linked the matrix the higher viscous it is, which kinetically inhibits the denaturation of the alpha-helices of the hair.

Differential scanning calorimetry or DSC is a thermoanalytical technique in which the difference in the amount of heat required to increase the temperature of a sample is measured as a function of temperature. The technic of DSC measurements for the investigation of human is for example described in Investigations of cosmetically treated human hair by differential scanning calorimetry in water, F. Wortmann, C. Springob, G. Sendelbach, Journal of Cosmetic Science, 53, 219-228, July/Aug 2002.

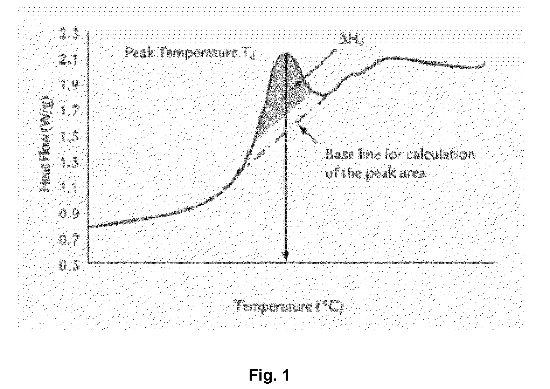

The denaturation temperature for the alpha helices of the hair is further illustrated by the enclosed figure 1.

The diagram in figure 1 shows on the x-axis the temperature in °C and on the y-axis the heat flow in W/g. The denaturation temperature is shown by the peak temperature T_{d}.

Damage caused to the hair was assessed by a FTIR (Fourier Transform Infrared) method, which was established to be suitable for studying the effects of keratin surface damage. Strassburger, J., J. Soc. Cosmet Chem., 36, 61 -74 (1985); Joy, M. & Lewis, D.M., Int. J. Cosmet. Sci., 13, 249-261 (1991); Signori, V. and Lewis, D.M., Int. J. Cosmet. Sci., 19, 1-13 (1997)). In particular, these authors have shown that the method was suitable for quantifying the amount of cysteic acid. In general, the oxidation of cystine is thought to be a suitable marker by which to monitor the overall oxidation of the keratinous part of the fiber. Net, the measurement of cysteic acid units by FT-IR was commonly used.

Signori and Lewis (D.M., Int. J. Cosmet. Sci., 19, 1-13 (1997)) showed that FT-IR using a diamond Attenuated Total Internal Reflection (ATR) cell was a sensitive and reproducible way of measuring the cysteic acid content of single fibers and bundles. Hence, the method that was employed to measure the cysteic acid content of multiple fibre bundles and full hair switches, was based upon the FTIR diamond cell ATR method employed by Signori and Lewis (1997). The detailed description of the method for testing the different damage inhibitors follows thereafter:

A Perkin Elmer Spectrum^{®} 1 Fourier Transform Infrared (FTIR) composition equipped with a diamond Attenuated Total Internal Reflection (ATR) cell was used to measure the cysteic acid concentration in mammalian or synthetic hair. In this method, hair switches of various sizes and colours were used. The switches were platted (~1 plait per cm) in order to minimize variations in surface area of contact between readings. The Oxidative hair Treatment Protocol described above was repeated for 5 cycles to mimic the behaviour of hair after repeated bleaching cycles. Following this treatment, four readings per switch were taken (1/3 and 2/3s down the switch on both sides), and an average calculated. Backgrounds were collected every 4 readings, and an ATR cell pressure of 1 N/m was employed. The cell was cleaned with ethanol between each reading, and a contamination check was performed using the monitor ratio mode of the instrument. As prescribed by Signori & Lewis in 1997, a normalized double derivative analysis routine was used. The original spectra was initially converted to absorbance, before being normalized to the 1450 cm⁻¹ band (the characteristic and invariant protein CH₂ stretch). This normalized absorbance was then twice derivatised using a 13-point averaging. The value of the 1450 cm⁻¹ normalized 2nd derivative of the absorbance at 1040 cm⁻¹ was taken as the relative concentration of cysteic acid. This figure was multiplied by -1x10⁴ to recast it into suitable units.

*The following tresses were used:*
Level 7 hair tresses (Kerling details) which were 3 times pre bleached (hereinafter tresses_1) and 5 times pre bleached (hereinafter tresses_2) were used to test the pretreatment compositions.

*The following pretreatment compositions were used:*

**Table 1**

| Ingredients | PC_1 | PC_2* |
|---|---|---|
| Malic acid [wt%] | 1 | |
| Citric acid [wt%] | | 0,3 |
| DTPMP [wt%] | 3 | |
| EDDS [wt%] | | 3 |
| Water [wt%] | q.s | q.s |
| Propylene Glycol [wt%] | 2.8 | 2.8 |
| Hydroxyethylcellulose [wt%] | 1.5 | 1.5 |
| Quaternium-80 [wt%] | 2 | 2 |
| EDTA [wt%] | 0.1 | 0.1 |
| Cocamidopropyl Betaine [wt%] | 2.5 | 2.5 |
| PEG-40 Hydrogenated Castor Oil [wt%] | 0.3 | 0.3 |

| | | |
|---|---|---|
| *not according to the invention | | |

The pH value of the composition according to the invention (PC_1) was 3.97 the pH value of composition PC_2* was 3.82.

The storage stability of the composition according to the invention (PC_1) was evaluated. The initial pH value was measured directly after the preparation of composition PC_1. Subsequently a 150 ml sample was stored in a sealed wide-neck bottle in a heating cabinet at 40°C. The pH value was measured after 1 month and after 3 months. The pH values were measured using a pH-meter from Mettler. The results are shown it the table below.

| | |
|---|---|
| pH initial | 3.97 |
| pH value after 1 month 40°C | 3.96 |
| pH value after 3 month 40°C | 3.99 |

Malic acid (Component (A)) was obtained by Active Concepts Distribution LLC in form of the racemic pre acid.

Citric acid was obtained by Cargill in form of the free acid.

DTPMP (Component (B)) was obtained by Zschimmer & Scharz in form of the heptasaodium salt.

EDDS was obtained by Innospec in form of the trisodium salt.

The denaturation temperatures (Td) of the tresses_1 and tresses_2 were determined via DSC. To the tresses_1 and tresses_2 the pretreatment composition (PC_1) was applied for 15 minutes and the denaturation temperatures (Td) of the pretreated tresses (tresses_1_PC_1 and tresses_2_PC_1) were also determined via DSC. The denaturation temperatures (T_{d}) are shown in table 2.

**Table 2**

| | tresses_1 | tresses_1_PC_1 | tresses_2 | tresses_2_PC_1 |
|---|---|---|---|---|
| (T_{d}) [°C] | 144,35 | 148,10 | 140,15 | 144,30 |

The results in table 2 proof that the denaturation temperatures (T_{d}) of the pretreated tresses_1_PC_1 rise by 3.75 °C the denaturation temperatures (T_{d}) of the pretreated tresses_2_PC_1 rises by 4.15 °C.

Further examples were conducted, wherein the tresses_1 were treated with an oxidative hair colouring composition with or without applying a pretreatment composition.

For hair colouring an oxidative hair colouring composition of Wella was used. The oxidative hair colouring composition was obtained by mixing the test tint composition and the oxidative composition show in the next tables.

| Test tint composition | |
|---|---|
| DI water [wt%] | q.s. |
| Disodium EDTA (BASF) [wt%] | 0.100 |
| Ascorbic acid (OSKAR BERG) [wt%] | 0.300 |
| Sodium Sulfite (BCD CHEMIE) [wt%] | 0.400 |
| Sodium Diethylenetriamine Pentamethylene Phosphonate (25% in water) [wt%] | 4.000 |
| Trisodium Ethylenediamine Disuccinate (38% in water) [wt%] | 3.350 |
| Monoethanolamine (MEA) (SASSOL) [wt%] | 10.000 |
| Citric acid (Cargill) [wt%] | 1.5 |
| **Crème pre-mix formula** | **40.000** |
| Cetearyl alcohol (BASF) [wt%] | (10.000) |
| Steareth-20 (CRODA) [wt%] | (2.000) |
| DI water [wt%] | (27.000) |

| Oxidative Composition (Developer 9%) | |
|---|---|
| DI water [wt%] | q.s. |
| Salicylic acid [wt%] | 0.100 |
| Disodium phosphate [wt%] | 0.080 |
| Phosphoric acid (85%) | 0.060 |
| Etidronic acid [wt%] | 0.010 |
| Hydrogen peroxide (50% Hydrogen peroxide Interox co-SO, SOLVAY) [wt%] | 18.000 |
| **Crème pre-mix formula** [wt%] | **20.000** |
| Cetearyl alcohol (BASF) [wt%] | (5.000) |
| Steareth-20 (CRODA) [wt%] | (1.500) |
| DI water [wt%] | (13.500) |

The formulations were prepared according to the following methods.

Crème pre-mix formula was a part of the tint composition and the oxidizing composition. A crème pre-mix is created by combining together DI water (68.5%), cetearyl alcohol (25%) and Steareth-20 (6.5%). The mixture was heated to 80-85 °C to melt the waxes and surfactant with stirring. The composition was homogenized for 5 minutes and then cooling was started to reduce the product to room temperature.

### Tint compositions:

The remaining components of the tint composition were mixed together at room temperature with DI water. When all of the materials were dissolved, 40 wt.% of the crème pre-mix was added, and the mixture stirred until a uniform consistency was obtained. The product was then stored in an aluminium tube until used.

### Oxidizing compositions:

The remaining components were mixed together with the DI water at room temperature. When all of the materials were dissolved, 20 wt.% of the crème pre-mix was added, and the mixture stirred until a uniform consistency was obtained. The product was then stored in a plastic bottle until used.

One part of the tint composition was mixed with 1.5 parts of oxidising composition (Welloxon Perfect 9%) to obtain the oxidative hair colouring composition.

To tresses_1 the oxidative hair colouring composition was applied. Three examples were conducted in the first example (tresses_1_col*) the tresses_1 were coloured without applying a pretreatment composition. In the second example (tresses_1_PC2*_col) before colouring the tresses_1 were treated with the non-inventive pretreatment composition PC2* for 10 minutes. In the third example (tresses_1_PC1_col) before colouring the tresses_1 were treated with the inventive pretreatment composition PC1 for 10 minutes. The oxidative hair colouring composition in each example was applied for 35 minutes.

The denaturation temperatures (T_{d}) are shown in table 3.

**Table 3**

| | tresses_1_col* | tresses_1_PC2*_col | tresses_1_PC1_col |
|---|---|---|---|
| (T_{d}) [°C] | 136,63 | 137,57 | 137,73 |

| | | | |
|---|---|---|---|
| *not according to the invention | | | |

The tresses pretreated with the inventive pretreatment composition (tresses_1_PC1_col) show a higher denaturation temperature (T_{d}) than the tresses treated with the non-inventive pretreatment composition (tresses_1_PC2*_col) and the non-pretreated tresses (tresses_1_col*) the temperature rise is 0.16 °C and 1.1 °C respectively.

The damage results are shown in table 4.

**Table 4**

| Example | ΔFT-IR Damage¹ | Statistical Groups | |
|---|---|---|---|
| tresses_1_col* | 71.64 | B | |
| tresses_1_PC1_col | 66.71 | | C |

| | | | |
|---|---|---|---|
| 1) Δ refers to the difference between the measurement of a PC1-treated versus an PC1-untreated tress. | | | |

The tresses pretreated with the inventive pretreatment composition (tresses_1_PC1_col) show less damage versus tress_1_col.

## Claims

1. A composition (PC) comprising as components
(A) malic acid and/or malic acid salt(s),
(B) at least one chelant comprising diethylenetriamine penta(methylene phosphonic acid) and/or diethylenetriamine penta(methylene phosphonic acid) salt(s),
(C) at least one thickener,
(D) at least one cosmetically acceptable solvent,
(E) at least one surface active ingredient, and
(F) optionally, at least one care ingredient,
wherein the composition (PC) has a pH value in the range of 2.5 to 6.5.

2. The composition (PC) according to claim 1, comprising
component (A) in an amount of from 0.1 to 2 % by weight,
component (B) in an amount of from 0.1 to 1.5 % by weight,
component (C) in an amount of from 0.1 to 5 % by weight, and
component (D) in an amount of from 71.75 to 98.7 % by weight,
component (E) in an amount of from 1 to 5 % by weight,
component (F) in an amount of from 0 to 5 % by weight,
wherein the % by weight values are based on the total weight of the composition (PC).

3. The composition (PC) according to any of claims 1 to 2, wherein the composition (PC) has a viscosity in the range of 1000 to 2000 mPas, measured on a Brookfield RS viscosimeter, cone C75, shear rate 64.5 per second, at 25 °C, 30 seconds spin time.

4. The composition (PC) according to any of claims 1 to 3, wherein component (C) is at least one water soluble polymer preferably selected from the group consisting of cellulose-based polymers, polysaccharide-based polymers, and acrylate-based polymers.

5. The use of a composition (PC) as defined in any of claims 1 to 4, as a pretreatment composition for a colouring or bleaching process.

6. The use of a composition (PC) as defined in any of claims 1 to 4, for protecting keratin fibers during a colouring or bleaching process.

7. A process for colouring or bleaching keratin fibers comprising the following steps
i) applying to the keratin fibers a composition (PC) as defined in any of claims 1 to 4,
ii) applying to the keratin fibers a colouring or a bleaching composition.

8. The process according to claim 7, wherein the colouring or the bleaching composition comprises at least one oxidizing agent (HD) selected from the group consisting of hydrogen peroxide, urea peroxide, alkali metal bromates, peroxygenated salts, peracids, percarbonates, persulfates and precursors thereof, and mixtures thereof.

9. The process according to claim 7 or 8, wherein the composition applied during step ii) is an oxidative hair colouring composition (HC) comprising as components
(HA) at least one oxidative coupler dye precursor,
(HB) at least one oxidative primary dye precursor,
(HC) at least one alkalizing agent, and
(HD) at least one oxidizing agent.

10. The process according to any of claims 7 to 9, wherein in step i) the composition (PC) is left on the keratin fibers for a time ranging from 1 to 60 min.

11. The process according to any of claims 7 to 10, wherein the process does not comprise a rinsing step between step i) and step ii).

12. A kit comprising the composition (PC) according to any of claims 1 to 4, and the oxidative hair colouring composition (HC) as defined in claim 9, and optionally a conditioner.

13. The kit according to claim 12, wherein the kit comprises the oxidative hair colouring composition (HC) in form of a tint composition comprising components (HA), (HB), and (HC), and an oxidizing composition comprising component (HD), wherein the tint composition and the oxidizing composition can be mixed together to obtain the hair colouring composition (HC).

14. A multi compartment device comprising
- a first compartment containing the composition (PC) according to any of claims 1 to 4;
- a second compartment containing an oxidizing composition comprising as component (HD) at least one oxidizing agent,
- optionally a third compartment containing a tint composition comprising as components (HA) at least one oxidative coupler dye precursor, (HB) at least one oxidative primary dye precursor, and (HC) at least one alkalizing agent, and
- optionally a fourth compartment comprising a conditioner.
